# FASCICULE DE BREVET EUROPEEN

(11) **EP 0 841 893 B1**
(45) Date de publication et mention de la délivrance du brevet: **26.09.2001**
(21) Numéro de dépôt: 96925814.4
(22) Date de dépôt: 18.07.1996
(51) Int. Cl.: A61K 7/06, A61K 7/48

(54) **ASSOCIATION SALICYLATE DE ZINC ET EXTRAIT DE SERENOA REPENS POUR LE TRAITEMENT DE LA SEBORRHEE ET COMPOSITIONS DERMO-COSMETIQUES LA COMPRENANT**
KOMBINATION EINES ZINKSALICYLATS UND EINES SERENOA REPENS-EXTRAKTS ZUR BEHANDLUNG DER SEBORRHÖ UND DIESE ENTHALTENDE KOSMETISCHE HAUTPFLEGEMITTEL
COMBINATION OF ZINC SALICYLATE AND A SERENOA REPENS EXTRACT FOR TREATING SEBORRHOEA, AND COSMETIC SKIN CARE COMPOSITIONS CONTAINING SAME

(30) Priorité: 20.07.1995 FR 9508792
(43) Date de publication de la demande: 20.05.1998
(73) Titulaire: Pierre Fabre Dermo-Cosmetique, 92100 Boulogne (FR)
(72) Inventeur: JEANJEAN, Michel, F-31370 Castanet-Tolosan (FR); NAVARRO, Roger, F-09100 Pamiers (FR)
(74) Mandataire: Ahner, Francis
(86) Numéro de dépôt international: FR9601127
(87) Numéro de publication internationale: WO9703639

(56) Documents cités:
- WO-A-91/02525
- FR-A- 2 556 968
- FR-A- 2 643 375
- FR-A- 2 665 637
- US-A- 3 821 370

## Description

La présente invention se rapporte à une composition, utile en dermatologie et cosmétologie, permettant de lutter contre la séborrhée de la peau et plus particulièrement du cuir chevelu.

Aujourd'hui, l'augmentation de la séborrhée du cuir chevelu, à l'origine de l'apparition des cheveux gras, a cessé d'être un problème purement dermatologique pour devenir un phénomène de société. Plus un désagrément qu'une maladie au sens propre, il constitue une atteinte à l'intégrité de l'image corporelle mal vécue au quotidien par le patient.

Pour mieux saisir les données de l'invention, il convient de souligner quelques points forts de l'étiologie de la séborrhée.

On peut la définir comme étant un traceur de l'activité de la glande sébacée, située à proximité du follicule pileux. La séborrhée est un phénomène physiologique se traduisant par la fabrication puis l'excrétion du sébum. Lorsque cette dernière est excessive, elle entraîne l'apparition des cheveux gras. C'est ce qu'il est convenu d'appeler l'hyperséborrhée. Elle est la conséquence d'un dérèglement du fonctionnement de la glande sébacée.

Plusieurs causes sont à l'origine de ce disfonctionnement, les principales étant d'ordre biochimique ou hormonal. Ainsi, la dérégulation de la sébogénèse, la péroxydation des lipides et le déséquilibre de la flore microbienne sont directement responsables de l'hyperséborrhée.

Le contrôle de la sécrétion sébacée est donc extrêmement subtil, faisant appel à de nombreux facteurs dont les plus importants sont hormonaux. C'est, par conséquent, un disfonctionnement endocrinien qui exacerbe la fonction séborrhéique.

C'est directement sur la glande sébacée, considérée comme un récepteur hormonal spécifique que se fait cette stimulation excessive androgénique. La testostérone présente dans le sang sous forme libre est le point de départ. Lorsqu'elle atteint le cytoplasme de la cellule sébacée, elle s'y transforme en dihydrotestostérone (DHT) sous l'action d'un enzyme spécifique, la 5-α-réductase. Sous cette forme activée, l'hormone DHT pénètre dans le noyau de la cellule où elle stimule l'ADN, induisant ainsi une accélération de la multiplication cellulaire. Il en résulte un développement exagéré de la glande sébacée et un accroissement de débit du sébum entraînant un excès de séborrhée. Parallèlement, cet état séborrhéique constitue un terrain favorable à la prolifération de bactéries lipiphiles comme le pityrosporum ovale. Douées d'une activité enzymatique lipolytique, elles sécrètent des lipases et hydrolysent les triglycérides du sébum.

Parmi les acides gras libérés, certains s'avèrent irritants, induisant une inflammation périfolliculaire et une hypertrophie de la glande sébacée dont le débit d'excrétion est augmenté.

Les moyens de traitement dont le patient dispose à ce jour sont essentiellement des shampoings traditionnels n'agissant qu'en surface. En effet, la plupart des shampooings actuels ont une fonction passive. Ils agissent comme de simples nettoyants de la tige pilaire, conférant aux cheveux propreté et esthétique mais sont sans action sur le site même de la glande sébacée. Les principes actifs les plus utilisés dans les shampooings pour cheveux gras sont à l'origine d'une action asséchante avec le soufre ou des dérivés soufrés ou d'une action astringente apportée par des extraits végétaux comme l'hamamelis ou certaines saponines.

Néanmoins, beaucoup de produits proposés restent déceptifs, la plupart en raison d'une action dégraissante trop importante, induisant un effet rebond par excitation de la glande sébacée. Dans la majorité des cas, cela est vécu par le consommateur comme une absence de réponse au traitement.

La présente invention concerne un produit de combinaison qui permet de lutter contre la séborrhée par une double action, d'une part sur le site même de la glande sébacée par une action directe sur la 5-α-réductase enzyme responsable du désordre endrocrinien, et d'autre part sur la composante inflammatoire induite par la séborrhée.

On a constaté que ces deux niveaux d'activité conjugués sont indispensables pour obtenir des résultats satisfaisants se traduisant par une diminution de la séborrhée et un retard dans son apparition.

Le produit selon l'invention consiste en l'association de deux principes actifs agissant en complémentarité et en synergie.

Le premier principe actif est un extrait lipostérolique de serenoa repens qui bloque l'activité enzymatique de la 5-α-réductase.

Le second principe actif est le salicylate de zinc qui agit sur la composante inflammatoire induite.

Comme extrait lipostérolique de serenoa repens, on emploie avantageusement l'extrait obtenu par le procédé décrit dans la demande de brevet français N° 2 643 375.

L'extrait lipostérolique est particulièrement riche en composés polycycliques dont la structure de type stérolique est proche de celle de la dihydrotestostérone. Il en résulte une compétition chimique avec l'hormone responsable, se traduisant par une diminution de l'activité enzymatique de la 5-α-réductase, donc de la sébogénèse. Par ailleurs, l'extrait lipostérolique de serenoa repens peut être enrichi en acides gras insaturés en C18 appartenant à la famille des acides gras essentiels tels que l'acide octadécénoïque et l'acide linoléique lui conférant des propriétés de transport et de pénétration transmembranaire, particulièrement intéressantes pour la vectorisation de l'activité.

D'une manière avantageuse, le rapport pondéral de l'extrait de serenoa repens par rapport au salicylate de zinc est compris entre 0,01 et 100, de préférence compris entre environ 0,1 et environ 10, de manière plus préférentielle entre environ 0,5 et environ 1.

La présente invention concerne également une composition dermo-cosmétique comprenant le produit de combinaison décrit plus haut à titre de composant actif et un véhicule approprié pour une application topique, en particulier sous la forme de lotions, shampooings, savons liquides, gels, savons ou pains dermatologiques, ou encore de type émulsions (laits, crèmes, pommades, mousses aérosols).

La composition selon l'invention comprend de préférence entre 0,05 et 5 % en poids d'extrait lipostérolique de serenoa repens et entre 0,1 et 5 % en poids de salicylate de zinc.

La composition selon l'invention permet l'administration simultanée des deux composants actifs du produit de combinaison selon l'invention.

Toutefois, ces deux composants actifs pourront également être employés en association séparément ou encore décalés dans le temps.

Un même rapport pondéral préférentiel entre les deux composants, tel que défini pour le produit de combinaison, sera recherché pour leur usage séparé ou décalé dans le temps.

Outre les effets bénéfiques complémentaires décrits précédemment, il a été constaté que les deux principes actifs, extrait lipostérolique de serenoa repens et salicylate de zinc agissaient en synergie et permettaient de diminuer les sécrétions de sébum et de retarder de façon très significative le regraissage des cheveux. Cette constatation inattendue et pour le moins originale nous permet d'accéder à un niveau d'activité particulièrement intéressant dans le produit proposé.

L'activité synergique des deux principes actifs associés à été mise en évidence par un test d'usage conduit pendant 6 semaines sur des sujets volontaires selon le protocole défini comme suite.

Les sujets sélectionnés appartiennent aux deux sexes (environ 60 % d'hommes et 40 % de femmes) d'âges compris entre 20 et 40 ans, s'identifiant comme atteints d'hyperséborrhgée, les sujets reconnaissant se laver les cheveux tous les jours. Tout sujet ayant un traitement capillaire concomittant a été éliminé ainsi que tout cas d'hyperséborrhée d'origine connue (médimenteuse ou dépressive). La durée du test a été fixée à 6 semaines et les produits appliqués un jour sur deux.

Trois groupes de sujets ont été constitués :
- le groupe GT constitué de 42 personnes a reçu un shampooing selon l'invention renfermant les deux principes actifs précités, à savoir l'extrait lipostérolique de serenoa repens décrit dans la demande de brevet FR 2 643 375 et le salicylate de zinc ;
- le groupe GZ représenté par 37 sujets a reçu un shampooing ne renfermant que le salicylate de zinc ;
- le groupe GS représenté par 33 sujets a reçu un shampooing ne renfermant que l'extrait lipostérolique de serenoa repens décrit dans la demande de brevet FR 2 643 375.

Un bilan a été effectué au bout de 2 semaines et 6 semaines. Les résultats sont exprimés par les cotations suivantes recueillies par interrogatoire auprès de chaque sujet :
A : Aucun résultat.
B : Diminution perceptible de la séborrhée.
C : Diminution jugée importante (cuir chevelu non gras).
D : Retour à un état dit normal.

Cette cotation tient compte du regraissage du cheveu tel qu'il est perçu par chaque sujet. Les résultats comparatifs relatifs à l'analyse des trois groupes GT, GZ et GS sont exprimés dans le tableau suivant en pourcentage de chaque cotation aux temps T0 + 2 semaines et T0 + 6 semaines.

| Cotations exprimées en % | Lecture à 2 semaines de traitement | | | Lecture à 6 semaines de traitement | | |
|---|---|---|---|---|---|---|
| | Groupe GZ | Groupe GS | Groupe GT | Groupe GZ | Groupe GS | Groupe GT |
| A | 3 | 3,7 | 3 | 2,8 | 3,1 | 2,3 |
| B | 87 | 85,4 | 81,2 | 72,3 | 73,2 | 51,5 |
| C | 8,8 | 9,1 | 12,7 | 19,6 | 16,3 | 26,5 |
| D | 1,2 | 1,8 | 3,1 | 5,3 | 7,4 | 19,7 |

### Commentaires des résultats

Le tableau montre de façon significative que le groupe GT ayant été traité par le shampooing renfermant les deux principes actifs présente le meilleur score sur les deux critères :
- diminution jugée importante (C)
- retour à létat dit normal (D).

En effet, au bout de 6 semaines de traitement, le pourcentage total cumulé des avis favorables émis sur les critères C et D est de :
Groupe GZ = 24,9 %
Groupe GS = 23,7 %
Groupe GT = 46,2 %.

Le résultat est plus particulièrement significatif après 6 semaines de traitement lorsque l'on considère les avis favorables émis sur le critère D, avec 19,7 % pour le groupe GT contre 5,3 % pour le groupe GZ et 7,49 % pour le groupe GS.

Enfin, si l'on observe l'évolution des trois groupes entre la deuxième et la sixième semaine de traitement, on observe une diminution des pourcentages cumulés des avis A et B comme suit :
Groupe GZ = - 14,9%
Groupe GS = - 12,8 %
Groupe GT = - 30,4 %.

L'examen de ces résultats permet de conclure de façon significative à l'intérêt de l'association synergique dans laquelle le serenoa repens, doté d'une activité inhibitrice de la 5-α-réductase, voit son efficacité augmentée par le salicylate de zinc, anti-inflammatoire ne possédant pas d'action comparable.

### Exemples de formulation

L'excipient vecteur des principes actifs devra posséder une dermotolérance parfaite et être non sébogène. A ce titre, les agents nettoyants tensio-actifs seront choisis parmi ceux ne présentant pas d'activité stimulatrice sur la sébogénèse, donc ayant la plus grande neutralité vis-à-vis du complexe pilosébacé.

A titre d'exemple, nous citerons une formule de shampooing type répondant aux critères précédemment évoqués, comme illustration de produits rincés et une formule de lotion non rincée.

### Formule de shampooing

| | |
|---|---|
| Salicylate de zinc | 2 % |
| Extrait lipostérolique de serenoa repens | 1 % |
| Alkyl polyglucoside | 2,8 % |
| Alkyl éthoxy sulfocuccinate sodique | 7,5 % |
| Lipoprotéine végétale | 3,5 % |
| Polymère quaternaire volumateur démêlant | 1 % |
| Agent de viscosage | QS |
| Parfum - Agent conservateur | QS |
| Eau purifiée QSP | 100 ml |
| pH ajusté entre 4 et 4,5. | |

### Formule de lotion topique

La lotion capillaire peut être un exemple de formulation particulièrement adaptée à la vectorisation des principes actifs évoqués précédemment. En effet, en raison du milieu très solvant, elle facilite l'acheminement et la pénétration des principes actifs au niveau de la glande sébacée et augmente ainsi leur rapidité d'action.

### Exemple de lotion

| | |
|---|---|
| Salicylate de zinc | 0,5 % |
| Extrait lipostérolique de serenoa repens | 0,5 % |
| N-hydroxyéthyl acétamide | 0,35 % |
| Diméthyl polysiloxane 1 CS | 20 % |
| Diméthoxyméthane | 20 % |
| Parfum | QS |
| Ethénol QSP | 100 ml. |

## Revendications

1. Produit de combinaison **caractérisé en ce qu'**il consiste en l'association d'une part d'un extrait lipostérolique de serenoa repens et d'autre part du salicylate de zinc.

2. Produit de combinaison selon la revendication 1, **caractérisé en ce que** le rapport pondéral extrait lipostérolique de serenoa repens / salicylate de zinc est compris entre 0,01 et 100, de préférence compris entre environ 0,1 et environ 10, de manière plus préférentielle compris entre environ 0,5 et environ 1.

3. Composition dermo-cosmétique **caractérisée en ce qu'**elle comprend le produit de combinaison selon l'une des revendicaitons 1 ou 2 à à titre de composant actif, et un véhicule approprié pour son application topique.

4. Composition selon la revendication 3, **caractérisée en ce qu'**elle comprend entre 0,05 et 5 % en poids d'extrait lipostérolique de serenoa repens et entre 0,1 et 5 % en poids de salicylate de zinc.

5. Produit contenant un extrait lipostérolique de serenoa repens et du salicylate de zinc, comme produit de combinaison pour une utilisation simultanée, séparée ou étalée dans le temps pour le traitement de la séborrhée.

## Claims

1. Composite product, **characterized in that** it consists of the combination, on the one hand, of a liposterolic Serenoa repens extract and, on the other hand, of zinc salicylate.

2. Composite product according to Claim 1, **characterized in that** the liposterolic Serenoa repens extract/zinc salicylate weight ratio is between 0.01 and 100, preferably between about 0.1 and about 10, more preferably between about 0.5 and about 1.

3. Cosmetic skin-care composition, **characterized in that** it comprises the composite product according to either of Claims 1 and 2 as active ingredient, and a suitable vehicle for the topical application thereof.

4. Composition according to Claim 3, **characterized in that** it comprises between 0.05 and 5% by weight of liposterolic Serenoa repens extract and between 0.1 and 5% by weight of zinc salicylate.

5. Product containing a liposterolic Serenoa repens extract and zinc salicylate, as composite product for use simultaneously, separately or spaced out over time for the treatment of seborrhoea.

## Patentansprüche

1. Kombinationsprodukt, **dadurch gekennzeichnet, daß** es aus einer Vereinigung einerseits eines liposterolischen Extraktes von Serenoa repens und andererseits von Zinksalicylat besteht.

2. Kombinationsprodukt nach Anspruch 1, **dadurch gekennzeichnet, daß** das Gewichtsverhältnis liposterolischer Extrakt von Serenoa repens/Zinksalicylat 0,01 bis 100, bevorzugt etwa 0,1 bis etwa 10, bevorzugter etwa 0,5 bis etwa 1 beträgt.

3. Dermokosmetische Zusammensetzung, **dadurch gekennzeichnet, daß** sie das Kombinationsprodukt nach einem der Ansprüche 1 oder 2 als aktiven Bestandteil und ein geeignetes Vehikel für seine topische Anwendung umfaßt.

4. Zusammensetzung nach Anspruch 3, **dadurch gekennzeichnet, daß** sie zwischen 0,05 und 5 Gew.-% liposterolischen Extrakt von Serenoa repens und 0,1 bis 5 Gew.-% Zinksalicylat umfaßt.

5. Produkt, enthaltend einen liposterolischen Extrakt von Serenoa repens und Zinksalicylat als Kombinationsprodukt für eine gleichzeitige, getrennte oder zeitlich gestaffelte Verwendung für die Behandlung von Seborrhoe.
